# EUROPEAN PATENT SPECIFICATION

(11) **EP 2 237 746 B1**
(45) Date of publication and mention of the grant of the patent: **04.05.2016**
(21) Application number: 08867381.9
(22) Date of filing: 19.12.2008
(51) Int. Cl.: A61F 2/24

(54) **PERCUTANEOUS HEART VALVE AND SYSTEM**
PERKUTANE HERZKLAPPE UND SYSTEM
VALVULE CARDIAQUE PERCUTANÉE ET SYSTÈME

(30) Priority: 28.12.2007 US 5881
(43) Date of publication of application: 13.10.2010
(73) Proprietor: Boston Scientific Limited, Hamilton HM11 (BM)
(72) Inventor: KVEEN, Graig, L., Maple Grove MN 55311 (US); JENSON, Mark, L., Greenfield MN 55357 (US); THIELEN, Joseph, M., Buffalo MN 55313 (US); DRASLER, William, J., Minnetonka MN 55345 (US); EIDENSCHINK, Tracee, E.J., Wayzata MN 55391 (US)
(74) Representative: Peterreins Schley
(86) International application number: PCT/US2008/013888
(87) International publication number: WO 2009/085206

(56) References cited:
- WO-A-2006/127765
- WO-A-2008/150529
- WO-A-2009/045338
- US-A1- 2005 234 546
- US-A1- 2006 149 360
- US-A1- 2006 259 136

## Description

### Technical Field

The present disclosure relates generally to devices for use in the cardiac system, and more particularly, to a device for native valve replacement and/or augmentation.

### Background

The circulatory system of mammals includes the heart and the interconnecting vessels throughout the body, including both veins and arteries. In particular, the human heart includes four chambers, the left and right atrium and the left and right ventricle. The mitral valve allows blood flow in one direction and is positioned between the left ventricle and the left atrium. Also, the tricuspid valve is positioned between the right ventricle and the right atrium, the aortic valve is positioned between the left ventricle and the aorta, and the pulmonary valve is positioned between the right ventricle and the pulmonary artery.

Each heart valve functions in concert to move blood throughout the circulatory system. As such, the right ventricle pumps oxygen-poor blood from the body to the lungs and then into the left atrium. From the left atrium, the blood is pumped into the left ventricle and then out through the aortic valve and into the aorta. The blood is then recirculated throughout the tissues and organs of the body and returns once again to the right atrium.

If the valves of the heart do not function properly, due either to disease or congenital defects, the circulation of the blood may be compromised. Diseased heart valves can be stenotic, where the valve does not open sufficiently to allow adequate forward flow of blood through the valve, and/or incompetent, where the valve does not close completely.

Incompetent heart valves can cause regurgitation or excessive backward flow of blood through the valve when the valve is closed. For example, certain diseases of the heart valves can result in dilation of the heart and one or more heart valves. When a heart valve annulus dilates, the valve leaflet geometry deforms and causes ineffective closure of the valve leaflets. The ineffective closure of the valve can cause regurgitation of the blood, accumulation of blood in the heart, and other problems.

US 2006/259136 A1 discloses a heart valve prosthesis having a self-expanding multilevel frame that supports a valve body comprising a skirt and plurality of leaflets.

### Summary

The present invention is directed at a percutaneous prosthetic heart valve as defined by the independent claims 1 and 6. Other embodiments are described in the dependent claims.

### Brief Description of the Drawings

Figure 1 illustrates a rolled out view of an embodiment of a prosthetic heart valve of the present disclosure.
Figure 2 illustrates a rolled out view of an embodiment of a stent anchoring frame according to the present disclosure.
Figure 3A illustrates a rolled out view of an embodiment of a stent anchoring frame and Figure 3B illustrates a plan view of an embodiment of a stent anchoring frame according to the present disclosure.
Figures 4A and 4B illustrate an embodiment of a valve frame according to the present disclosure.
Figure 5 illustrates a rolled out view of an embodiment of a valve frame 502 according to the present disclosure.
Figures 6A-6E illustrate an example of a system 658 , which does not form part of the invention.
Figures 7A-7C illustrate embodiments of a first retractable sheath according to the present disclosure.

### Detailed Description

Embodiments of the present disclosure are directed to a device and system, for percutaneous heart valve replacement. For example, the device can include a prosthetic heart valve that can be used to replace an incompetent valve (e.g., an aortic valve or a pulmonary valve) in a body lumen. Embodiments of the prosthetic valve include a valve frame, a valve leaflet coupled to the valve frame, and a stent anchoring frame coupled to the valve frame.

The prosthetic heart valve of the present disclosure can allow for the repositioning and/or removal of the prosthetic heart valve during the percutaneous delivery of the prosthetic heart valve to a treatment site. In addition, the prosthetic heart valve of the present disclosure is designed to prevent migration of the prosthetic heart valve once it is deployed in a body lumen. Since the prosthetic heart valve is for heart valve replacement, the design requirements can vary as compared to, for example, a venous valve. For example, the prosthetic heart valve is designed to withstand in situ pressures of more than thirteen thousand three hundred thirty (13,330) Pascal (100 torr) in the forward flow direction, and nearly thirty-three thousand three hundred thirty (33,330) Pascal (250 torr) in the reverse flow direction. In contrast, a prosthetic venous valve can be designed to withstand, for example, in situ pressure across a prosthetic venous valve that is approximately two thousand six hundred seventy (2,670) Pascal (20 torr). In addition, the prosthetic heart valve is also designed to accommodate mechanical forces and movements which are imposed by the tissues to which they are attached without damage and without migration or misalignment.

As such, the prosthetic heart valve of the present disclosure includes an expandable valve frame coupled to an expandable stent anchoring frame, and at least one valve leaflet coupled to the valve frame. As discussed herein, by coupling the valve frame to the stent anchoring frame, the valve frame can be positioned at a treatment site and the stent anchoring frame can prevent migration of the valve frame.

The valve frame includes valve frame members and the stent anchoring frame includes stent frame members defining a first portion and a second portion of the stent anchoring frame. The second portion of the stent anchoring frame has greater flexibility than the first portion. In addition, the first portion of the stent anchoring frame and the valve frame define a length, where the stent frame members and the valve frame members along the length provide a contiguous surface over which a delivery device can repeatedly slide over the length in its entirety in two longitudinal directions. Also, the delivery device can slide repeatedly over the length when the first portion and the valve frame are in a partially expanded state during delivery from the delivery device.

The configuration of the first portion forms a contiguous surface that allows for the repositioning and removal of the prosthetic heart valve if the valve frame is not in the desired position. For example, once the prosthetic heart valve is at a delivery site, the valve frame can be at least partially deployed, allowing the function of the valve frame and valve leaflets to be observed. If the valve frame is not in a satisfactory position, a sheath is able to advance over the contiguous surface of the first portion of the stent anchoring frame and the valve frame, compressing the valve frame to allow for the repositioning of the prosthetic heart valve.

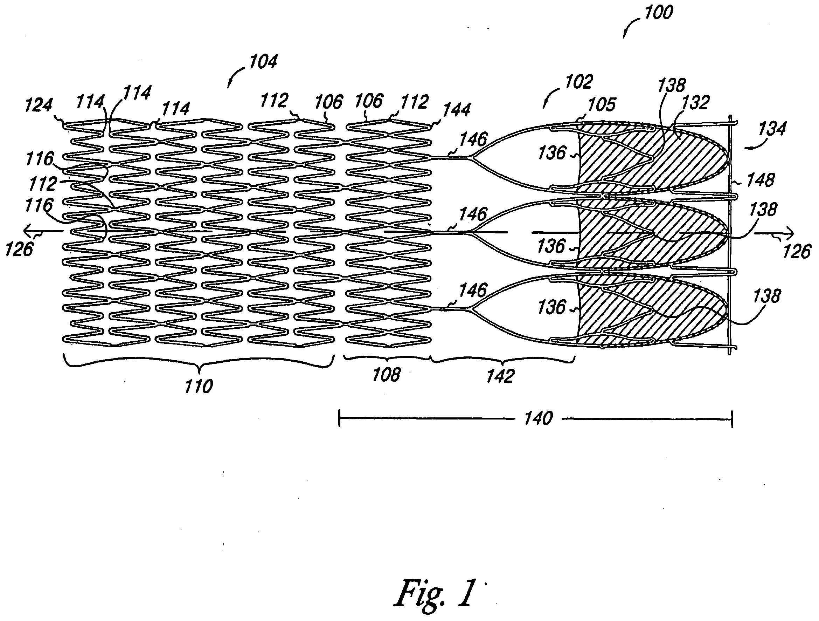

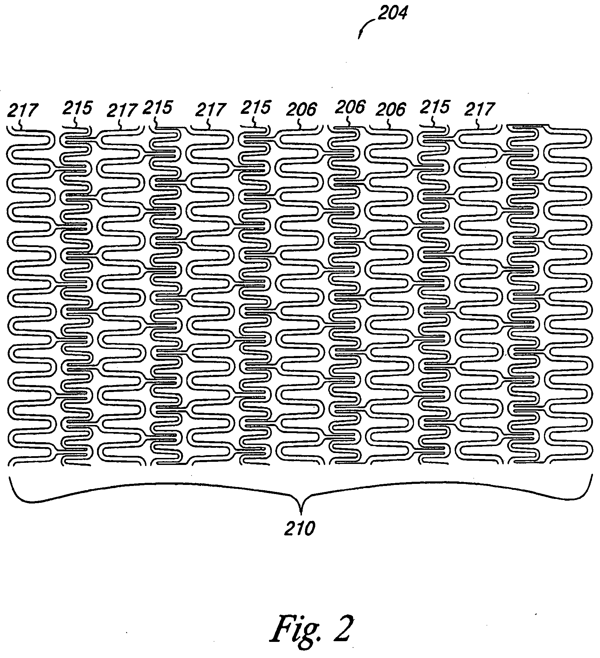

The figures herein follow a numbering convention in which the first digit or digits correspond to the drawing figure number and the remaining digits identify an element or component in the drawing. Similar elements or components between different figures may be identified by the use of similar digits. For example, 110 may reference element "10" in Fig. 1, and a similar element may be referenced as 210 in Fig. 2. As will be appreciated, elements shown in the various embodiments herein can be added, exchanged, and/or eliminated so as to provide any number of additional embodiments of valve and/or system. In addition, as will be appreciated, the proportion and the relative scale of the elements provided in the figures are intended to illustrate the embodiments of the present invention, and should not be taken in a limiting sense.

Various embodiments of the present disclosure are illustrated in the figures. Generally, the prosthetic heart valve can be implanted within the fluid passageway of a body lumen, for example, for replacement or augmentation of a valve structure within the body lumen (e.g., an aortic valve), to regulate the flow of a bodily fluid through the body lumen in a single direction.

The embodiments of the prosthetic heart valve of the present disclosure include a valve frame and a stent anchoring frame that self-expand. The valve frame and/or stent anchoring frame can self-expand to a fully deployed state and/or a semi-deployed state depending on what portions of the valve frame and/or stent anchoring frame are restrained by elements of a delivery device (e.g., sheaths). In some instances, the position of the prosthetic heart valve relative to the desired implant location can be adjusted to correct changes or misalignments of the heart valve that can occur during delivery. In addition, restraining portions of the valve frame and/or stent anchoring frame prior to completing the deployment can allow for adjustments due to movement caused by the flow output from the ventricle pushing on the deployment system, which can be the case when implanting, for example, an aortic valve.

As used herein, a semi-deployed state of the valve frame and/or stent anchoring frame lies between an undeployed state (i.e., the state of the valve frame and stent anchoring frame at the time the prosthetic valve is outside the body and in a delivery device) and a deployed state (i.e., the state of the valve frame and stent anchoring frame at the time the prosthetic valve is to be left in the body).

In the various embodiments, holding the valve frame in the deployed state while the stent anchoring frame is in the undeployed state allows the prosthetic heart valve to be positioned in a desired location prior to its final deployment. This staged deployment of the prosthetic heart valve of the present disclosure is in contrast to prosthetic heart valves that are deployed without the advantage of temporarily pausing at an intermediate deployment stage (i.e., the partial deployment state) to allow for adjustments in the placement of prosthetic heart valve prior to full deployment.

Figure 1 provides a rolled out view of an embodiment of a prosthetic heart valve 100, or prosthetic valve, of the present disclosure. The prosthetic valve 100 of the present embodiment can have a generally cylindrical shape. In addition, in some embodiments, the prosthetic valve 100 can have a cross-sectional shape that is oval, circular, or elliptical. In some embodiments, the prosthetic valve 100 can be conical, bulbous, or flare outward, as discussed herein. The cross-sectional shape of the prosthetic valve 100 can be determined by the method used to form the prosthetic valve 100. For example, in some embodiments, the prosthetic valve 100 can be heat set over a mandrel with an ovular cross-sectional shape. In such embodiments, the prosthetic valve 100, when delivered, can have, for example, an ovular cross-sectional shape. The same method can be used to produce prosthetic valves 100 with other cross-sectional shapes or with varying cross-sectional shapes along the longitudinal length of the prosthetic valve.

In addition, the size of the prosthetic valve 100 can be varied in accordance with whether the prosthetic valve 100 is to be used to replace the aortic valve or the pulmonary valve, or when the prosthetic valve 100 is to be used as a supplementary valve to be positioned in the vasculature. These dimensions (e.g., 20-30 millimeters (mm)) can be readily determined by techniques known by those skilled in the art.

The prosthetic valve 1 00 of the present disclosure includes an expandable valve frame 1 02 and an expandable stent anchoring frame 104 coupled to the valve frame 102. In some embodiments, the valve frame 102 can be positioned distal to the stent anchoring frame 104, where the valve frame 102 is coupled to a distal end 144 of the stent anchoring frame 104. In some embodiments, the valve frame 102 and the stent anchoring frame 104 can be self-expandable. The valve frame 102, the stent anchoring frame 104, and/or portions of the valve frame 102 and stent anchoring frame 104 can also be balloon expandable. In various embodiments, the valve frame 102 can be self-expandable while the stent anchoring frame 104 can be balloon expandable. Other configurations are also possible.

Examples of self-expanding frames include those formed from temperature-sensitive memory alloy which change shape at a designated temperature or temperature range. Alternatively, the self-expanding frames can include those having a spring-bias. Examples of suitable materials include, but are not limited to, medical grade stainless steel (e.g., 316L), titanium, tantalum, platinum alloys, niobium alloys, cobalt alloys, alginate, or combinations thereof. Examples of shape-memory materials include shape memory plastics, polymers, and thermoplastic materials which are inert in the body. Shape memory alloys having superelastic properties generally made from ratios of nickel and titanium, commonly known as Nitinol, are also possible materials.

In addition, the valve frame 102 includes valve frame members 105 while the stent anchoring frame 104 includes stent frame members 106. For the various embodiments, the valve frame members 105 and the stent frame members 106 can have similar and/or different cross-sectional geometries along the prosthetic valve 100 length. The similarity and/or the differences in the cross-sectional geometries can be based on one or more desired functions to be elicited from each portion of the valve frame 102 and/ or the stent anchoring frame 104. Examples of cross-sectional geometries include rectangular, non-planar configuration, round (e.g., circular, oval, and/or elliptical), polygonal, arced, and tubular. Other cross-sectional geometries are possible.

The valve frame 102 and stent anchoring frame 104 of the present disclosure can include stent frame members 106 and valve frame members 105 that provide adequate radial stiffness when in the expanded, or deployed, state. Adequate radial stiffness includes enough stiffness to ensure that the valve frame 102 and stent anchoring frame 104 maintain a cylindrical shape.

As discussed herein, a prosthetic valve 100 implanted to replace, for example, an aortic valve, can experience a large amount of in situ pressure with the backflow as well as the inflow of fluid through the prosthetic valve 100. The stent anchoring frame 104 is provided to reduce the likelihood that the prosthetic valve 100 will migrate after the prosthetic valve 100 is delivered and implanted at a delivery site, as well as throughout the life of the prosthetic valve 100. The stent anchoring frame 104 also can provide the ability to reposition the prosthetic valve 100 during a delivery procedure.

The stent anchoring frame 104 stent frame members 106 define a first portion 108 and a second portion 110. The second portion 110 has greater flexibility than the first portion 108, as discussed further herein. The greater flexibility of the second portion 110 can help the stent anchoring frame 104 to conform to a treatment site (e.g., body lumen) in which it is placed. By conforming to the treatment site, the stent anchoring frame 104 can better anchor into the treatment site, as compared to a stent anchoring frame 104 with uniform but lesser flexibility, preventing the movement of the prosthetic valve 100 when in use.

The stent anchoring frame 104, as illustrated in Figure 1, can include annular stent frame members 106, where the stent frame members 106 are connected using connectors 112. As used herein, a "connector" is defined as a piece of material positioned between two stent frame members 106. In the embodiment shown in Figure 1, the connectors 112 are positioned between two apices 114 on adjacent stent frame members 106. As used herein, an "apex" 114 is defined as a vertex formed by the stent frame members 106. The connectors 112 can also be positioned at other locations besides apex 114 to apex 114.

The first portion 108 of the stent anchoring frame 104 can include connectors 112 positioned between each apex 114 formed by the stent frame members 106. By having the connectors 112 positioned between each apex 114 formed by the stent frame member 106 in the first portion 108, the first portion 108 of the stent anchoring frame 104 has a contiguous surface. As used herein, a "contiguous surface" is defined as a surface with no free apexes; in other words, each apex 114 is connected to an adjacent stent frame member 106 by a connector 112. For example, as shown in Figure 1, each apex 114 is connected to an adjacent apex 114 by a connector 112. As discussed herein, the contiguous surface can allow for the repositioning of the prosthetic valve 100 during the delivery procedure.

As illustrated in Figure 1, the first portion 108, can consist of two stent frame members 106 and connectors 112 positioned between each apex 114 formed by the two stent frame members 106. The connector 112 can be formed of the same material, or a different material, as the stent frame members 106.

In some embodiments, the first portion 108 can consist of more than two stent frame members 106. In addition, because the connectors 112 are positioned at each apex 114 formed by the two stent frame members 106, the first portion 108 of the stent anchoring frame 104 is relatively stiff, or inflexible, as compared to the second portion 110 of the stent anchoring frame 104, as discussed herein. In addition, because the connectors 112 are positioned at each apex 114 formed by the stent frame members 106, a force applied to the first portion 108 can transmit the load throughout the first portion 108 uniformly, as discussed herein.

The strength and/or flexibility of the first portion 108 and the second portion 110 of the stent anchoring frame 104, however, can be adjusted by, for example, adjusting the number of frame members 106. As used herein, the "strength" of the various portions of the prosthetic valve 100 is defined as the ability to resist strain and/or stress. As used herein, the "flexibility" of the various portions of the prosthetic valve 100 is defined as the capability of being bent without breaking and/or becoming permanently deformed.

As will be appreciated, as the number of stent frame members 106 is increased in the first portion 108, the flexibility can decrease and the strength can increase. In addition, the strength and/or flexibility can be adjusted by changing the thickness of the stent frame members 106 and/or by changing the cross-sectional shape of the stent frame members 106. For example, by decreasing the thickness of the stent frame members 106, the flexibility of the first portion 108 can increase while the strength can decrease. The strength and flexibility properties of the stent anchoring frame 104 can also be determined by the material forming the stent anchoring frame 104.

As discussed herein, the second portion 110 of the stent frame 104 has greater flexibility than the first portion 108. The difference in flexibility between the first portion 108 and second portion 110 can be accomplished in several ways. For example, as illustrated in Figure 1, in the first portion 108, the connectors 112 are positioned between each apex 114 formed by the stent frame member 106. In the second portion 110, the connectors 112 are positioned between less than each apex 114 formed by the stent frame member 106.

By having the connectors 112 positioned between each apex 114 formed by the stent frame member 106 in the first portion 108, the first portion 108 is less flexible than the second portion 110. In addition, having the connectors 112 positioned between less than each apex 114 formed by the stent frame members 106 in the second portion 110 produces a second portion 110 with free apices 116. As discussed herein, the contiguous surface of the first portion 108 allows for the repositionability of the prosthetic valve 100, however, once the second portion 110 of the stent anchoring frame 104 is allowed to expand, the free apices 116 of the second portion 110 can prevent any further repositioning or realignment.

In other embodiments, the flexibility of the second portion 110 can be modified by using a more flexible material in forming the stent frame members 106, and/or by forming the second portion 110 stent frame members 106 with a smaller cross-sectional diameter as compared to the first portion 108 stent frame members 106.

In some embodiments, the greater flexibility of the second portion 110 of the stent anchoring frame 104 can increase the likelihood that the stent anchoring frame 104 will conform to curves and/or irregularities in a surface of a body lumen. By conforming with the body lumen, the second portion 110 of the stent anchoring frame 104 can more aptly embed itself into the body lumen wall, preventing migration of the stent anchoring frame 104, and thereby the valve frame 102.

As shown in Figure 1, in some embodiments, the second portion 110 of the stent anchoring frame 104 is positioned proximal to the first portion 108 in a longitudinal direction. In addition, as will be appreciated, the second portion 110 is illustrated having five stent frame members 106, however, the second portion 110 can include more or less than five stent frame members 106.

Similar to the first portion 108 of the stent anchoring frame 104, the flexibility and/or strength of the second portion 110 can be adjusted by changing the thickness of the frame members 106, by changing the cross-sectional shape of the frame members 106, and/or by choosing a material with the desired flexibility and strength. In addition, the flexibility and/or strength can be tuned to the desired value by increasing or decreasing the number of connectors 112 between the apices 114 formed by the stent frame members 106. By increasing the number of connectors 112, the flexibility can be reduced, while the strength can be increased.

In some embodiments, the stent anchoring frame 104 can include a covering around at least a portion of the exterior surface of the stent anchoring frame 104. The covering can be formed of, for example, expanded polytetrafluoroethylene (ePTFE), or other materials.

As shown in Figure 1, the stent anchor frame 104 can have stent frame members 106 with a configuration where the apices 114 are formed into points. Other embodiments of the stent frame members 106 are also possible.

Figure 2 illustrates a rolled out view of an embodiment of a stent anchor frame 204. Figure 2 illustrates the second portion 210 of the stent anchor frame 204 having stent frame members 206 with different longitudinal flexibilities. As shown, the second portion 210 includes stent frame members 206 that alternate between a high flexibility stent frame member 215, and a low flexibility stent frame member 217. As used herein, the terms "high" and "low" refer to the degree of flexibility of the stent frame members 206 as compared to each other.

The high flexibility stent frame member 215 is a thinner stent frame member 206 and contains additional structural undulations as compared to the low flexibility stent frame member 217. By forming the high flexibility stent frame member 215 as such, both the high and low flexibility stent frame members 215, 217 can expand to the same radial dimension and have uniform radial strength along the length of the stent anchor frame 204. However, the high flexibility stent frame members 215 can have more flexibility in the longitudinal direction.

In addition, by including the alternating stent frame members 206 as shown in Figure 2, the second portion 210 can have increased flexibility in the longitudinal direction as compared to a second portion 210 without alternating stent frame members 206 (e.g., containing only low flexibility stent frame members 217). On the other hand, in some embodiments, the second portion 210 shown in Figure 2 can be formed with even greater flexibility when the second portion 210 contains high flexibility stent frame members 215 without the alternating design.

In addition, although not illustrated, one skilled in the art would appreciate that the second portion 210 of the stent anchoring frame 204 has greater flexibility than the first portion of the stent anchoring frame 204 when the first portion consists of low flexibility stent frame members 217.

As one skilled in the art will appreciate, Figure 2 is an example of how to adjust the flexibility of the stent anchoring frame 204. The stent frame members 206 can have different widths, frequencies of undulations, and/or materials to obtain a desired flexibility in the stent anchoring frame 204. In addition, this embodiment illustrates an approach to making the stent anchoring frame 204 more flexible without compromising the expandability or the radial strength of the stent anchoring frame 204.

In some embodiments, the configuration of high flexibility stent frame members 215 and low flexibility stent frame members 217 can be used to create a stent anchoring frame 204 with different diameters along the longitudinal length of the stent anchoring frame 204. In various embodiments, the length of one of the alternating stent frame members, for example, the high flexibility stent frame members 215 can be increased to cause the, for example, high flexibility stent frame members 215 to expand to a larger dimension as compared to the low flexibility stent frame members 217.

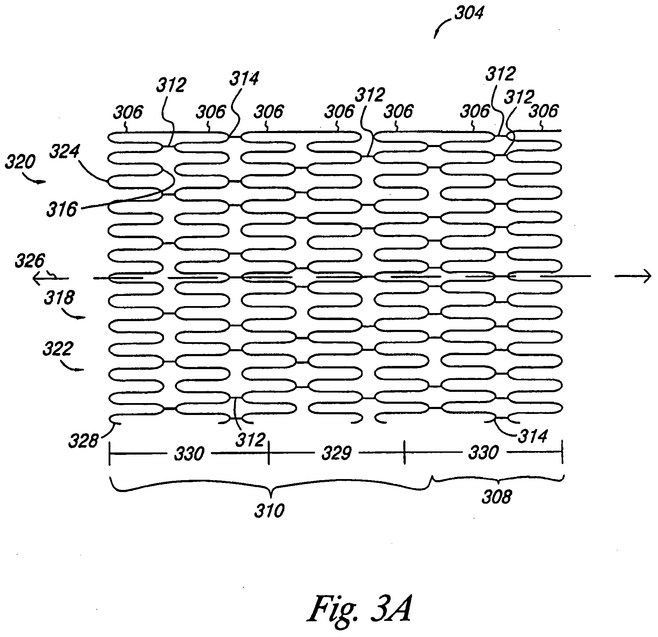

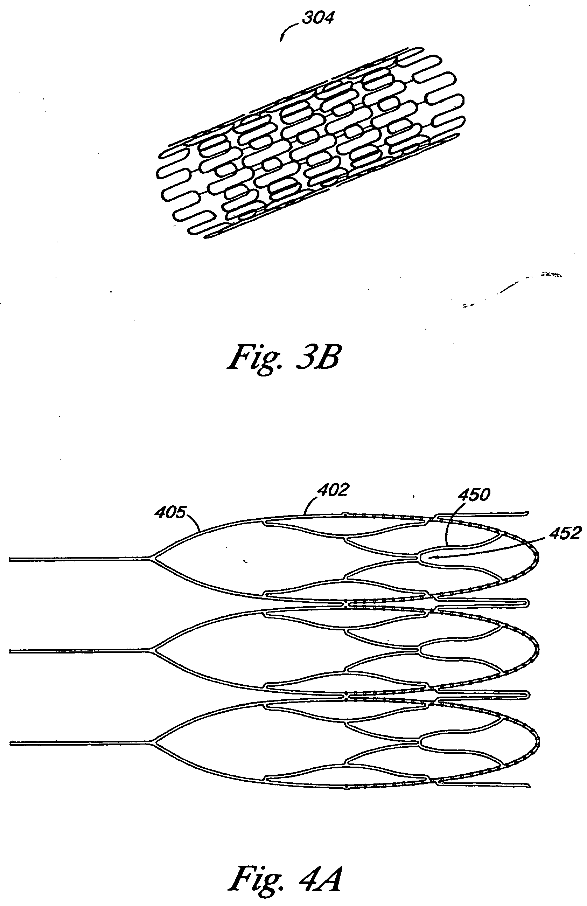

Figure 3A illustrates a rolled out view of an embodiment of a stent anchoring frame 304 and Figure 3B illustrates a plan view of an embodiment of a stent anchoring frame 304 according to the present disclosure. Figure 3A provides an example of a stent anchoring frame 304 where the apices 314 are curved. In addition, Figure 3A illustrates both the first portion 308 and the second portion 310. In this embodiment, the first portion 308 includes connectors 312 between each apex 314 formed by the stent frame members 306. The second portion 310, however, includes connectors 312 between less than each apex 314 formed by the stent frame members
306. By including connectors 312 between less than each apex 314, the second portion 310 can be more flexible than the first portion 308, as discussed herein.

As shown in Figure 3A, in some embodiments, connectors 312 can be included between certain apices 314 formed by the stent frame members 306, decreasing the flexibility of the second portion 310 in certain areas of the second portion 310 as compared to areas of the second portion 310 without connectors 312.

As shown in Figure 3A, the stent anchoring frame 304 second portion 310 includes an increased amount of connectors 312 in the middle 318 of the stent anchoring frame 304, and at the top 320 and bottom 322 of the stent anchoring frame 304, as compared to the portions of the stent anchoring frame 304 between the top 320 and middle 318 as well as the bottom 322 and middle 318 of the stent anchoring frame 304.

Since the stent anchoring frame 304 shown in Figure 3A is a rolled out view of the stent anchoring frame 304, one of ordinary skill in the art can appreciate that once the prosthetic valve is in a generally cylindrical form, the middle 318, and the top 320 and bottom 322 of the stent would be radially opposed to each other. In this embodiment, the increased amount of connectors 312 in the two radially opposing areas can cause the stent anchoring frame 304 to have less flexibility in the areas where there are an increased amount of connectors 312. Other connector configurations are also possible.

As discussed herein, the stent anchoring frame 304 can be generally cylindrical. In some embodiments, the proximal end 324 of the second portion 310 of the stent anchoring frame 304 can flare in a radially outward direction from a center axis 326 of the stent anchoring frame 304. In some embodiments, the most-proximal frame member 328 of the second portion 310 can expand to a deployed diameter larger than the deployed diameter of the other stent frame members 306. In various embodiments, more than one stent frame member 306 at the proximal end 324 of the second portion 310 can expand to a deployed diameter larger than the deployed diameter of the other stent frame members 306.

In some embodiments, portions of the stent anchoring frame 304 can expand to different diameters. For example, the stent anchoring frame 304 can have a middle portion 329 that expands to a larger diameter than two end portions 330, producing a bulbous shaped middle portion 329. On the other hand, the two end portions 330 can expand to a larger diameter than the middle portion 329. In addition, the stent anchoring frame 304 can expand to a different diameter than the valve frame included in the prosthetic heart valve. Other configurations are also possible.

In some embodiments, the different diameters can be accomplished by including stent frame members 306 formed of different materials, or of similar materials but with different post-processing. In various embodiments, stent frame members 306 having a longer length can be compressed to a greater degree as compared to stent frame members 306 with a shorter length, where the stent frame members 306 with a longer length can expand to a larger diameter once allowed to expand, as discussed herein with respect to Figure 2.

Referring back to Figure 1, as discussed herein, embodiments of the prosthetic valve 100 include a valve frame 102. The valve frame 102 includes valve leaflets 132 having surfaces defining a reversibly sealable opening for unidirectional flow of a liquid through the prosthetic valve 100. For example, the valve leaflets 132 can be coupled to the valve frame 102 so as to span and control fluid flow through the lumen of the prosthetic valve 100. For the present embodiment, the prosthetic valve 100 includes three valve leaflets 132 for a tri-leaflet configuration. As appreciated, mono-leaflet, bi-leaflet, and/or multi-leaflet configurations are also possible. Each of the valve leaflets 132 are coupled to the valve frame 102, where the leaflets 132 can repeatedly move between an open state and a closed state for unidirectional flow of a liquid through a lumen of the prosthetic valve 100.

As shown in Figure 1, the valve leaflets 132 can be coupled to the valve frame 102 at the distal end 134 of the valve frame 102, and extend to approximately the middle of the valve frame 102. As shown in Figure 1, the valve leaflets 132 can include a free edge 136 to move between a closed configuration and an open configuration to allow fluid to move through the prosthetic valve 100 while preventing backflow.

In one embodiment, the leaflets 132 can be derived from autologous, allogeneic, or xenograft material. As will be appreciated, sources for xenograft material (e.g., cardiac valves) include, but are not limited to, mammalian sources such as porcine, equine, bovine, and sheep. Additional biologic materials from which to form the valve leaflets 104 include, but are not limited to, explanted veins, pericardium, facia lata, harvested cardiac valves, bladder, vein wall, various collagen types, elastin, intestinal submucosa, and decellularized basement membrane materials, such as small intestine submucosa (SIS), amniotic tissue, or umbilical vein.

Alternatively, the leaflets 132 can be formed from a synthetic material. Possible synthetic materials include, but are not limited to, expanded polytetrafluoroethylene (ePTFE), polytetrafluoroethylene (PTFE), polystyrene-polyisobutylene-polystyrene (SIBS), polyurethane, segmented poly (carbonate-urethane), polyester, polyethlylene (PE), polyethylene terephthalate (PET), silk, urethane, Rayon, Silicone, or the like. In an additional embodiment, the synthetic material can also include metals, such as stainless steel (e.g., 316L) and nitinol. These synthetic materials can be in a woven, a knit, a cast, or other known physical fluid-impermeable or permeable configurations. In addition, plated metals (e.g., gold, platinum, rhodium) can be embedded in the leaflet 132 material (e.g., a sandwich configuration) to allow for visualization of the leaflets 132 post placement.

The leaflets 132 can also be formed of any combination of these exemplary materials, or these materials in combination with other materials, as are known in the art. A variety of known treatments and/or coatings can also be included in the leaflets 132.

The valve frame 102, as shown in Figure 1, includes valve frame members 105 forming the valve frame 102 and coupling the valve frame 102 to the stent anchoring frame 104. The valve frame members 105 can also provide a surface for leaflet 132 attachments as well as a surface to engage the body lumen wall when the prosthetic valve 100 is positioned at a treatment site.

As illustrated, the valve frame members 105 are coupled together such that all valve frame free edges 138 point in a direction towards the distal end 134 of the prosthetic valve 100. By forming the valve frame 102 so that all of the valve frame free edges 138 point in the direction towards the distal end 134 of the prosthetic valve 100, the first portion 108 of the stent anchoring frame 104 and the valve frame 102 define a length 140 of the prosthetic valve 100 that has a contiguous surface, as discussed herein. The contiguous surface allows a delivery device to repeatedly slide over the length 140 in its entirety in two longitudinal directions when the first portion 108 of the stent anchoring frame 104 and the valve frame 102 are in a partially expanded state during delivery from the delivery device, as discussed further herein.

As discussed herein, the valve frame 102 of the present disclosure can include valve frame members 105 that provide adequate radial stiffness when in the expanded, or deployed, state. Adequate radial stiffness includes enough stiffness to ensure that the valve frame 102 maintains a cylindrical shape, which ensures that the leaflets 132 close and open properly. Adequate radial stiffness can also ensure that there will be no parevalvular leakage, in other words, no leaking between the valve 100 and the aorta interface. Radial stiffness also can ensure that sufficient interaction between the prosthetic valve 100 and body lumen wall is provided so as to minimize the chance of prosthetic valve 100 migration as the prosthetic valve 100 closes and holds full body blood pressure.

In some embodiments, the valve frame 102 can include a transition zone 142, where the transition zone 142 is positioned between the valve leaflets 132 and the stent anchoring frame 104, and is coupled to a distal end 144 of the stent anchoring frame 104. As shown in Figure 1, in some embodiments, the transition zone 142 is positioned adjacent to the distal end 144 of the first portion 108 of the stent anchoring frame 104.

In various embodiments, the transition zone 142 can include transition zone members 146 coupled to the distal end 144 of the stent anchoring frame 104. The transition zone members 146 can be useful in distributing the load on the valve frame 102 to the stent anchoring frame 104 due to liquid going through the valve frame 102. As discussed herein, the first portion 108 of the stent anchoring frame 104 can include connectors 112 positioned at each apex 114 formed by the stent frame members 106. As liquid goes through the valve frame 102, the transition zone members 146 can transmit the load to the stent anchoring frame 104, where the configuration of the first portion 108 transmits the load throughout the first portion 108 uniformly. As such, the transition zone members 146 can be formed of a rigid material to keep the distance between the valve frame 102 and the stent anchoring frame 104 approximately static.

In some embodiments, the transition zone members 146 can be formed such that the transition zone members 146 have a high fatigue life, some compression strength, and high tensile strength. In some embodiments, the transition zone members 146 can be formed of a cable that is flexible in the radial direction but is inflexible in the longitudinal direction. In some embodiments, the transition zone members 146 can be formed of a braided member. In various embodiments, the transition zone members 146 can be formed of a cable around a solid core. As shown in Figure 1, the prosthetic valve 100 can include three transition zone members 146. Embodiments of the present disclosure, however, can include more or less than three transition zone members 146.

In some embodiments, the valve frame 102 can include a support ring 148 on the distal end 134 of the valve frame 102. Including the support ring 148 can reduce the amount of valve frame members 105 included in the valve frame 102. In addition, in some embodiments, the support ring 148 can flare in a radially outward direction from the center axis 126. In addition, embodiments of the valve frame 102 without the support ring 148 can include the distal end 134 of the valve frame 102 flared in a radially outward direction from the center axis 126.

In some embodiments, the valve frame 102 can include a fabric tube surrounding the exterior surface of the valve frame 102. The fabric tube can increase the likelihood that the valve frame 102 can form a fluid-tight seal at a body lumen wall. In other words, the fabric tube can prevent liquid from flowing around the valve frame 102 rather than through the valve frame 102.

The prosthetic valve 100 can further include one or more radio-opaque markers (e.g., tabs, sleeves, welds). For example, one or more portions of the valve frame 102 and/or stent anchoring frame 104 can be formed from a radio-opaque material. Radio-opaque markers can be attached to and/or coated onto one or more locations along the valve frame 102 and/or stent anchoring frame 104. Examples of radio-opaque materials include, but are not limited to, gold, tantalum, and platinum. The position of the one or more radio-opaque markers can be selected so as to provide information on the position, location, and orientation of the prosthetic valve 100 during its implantation.

As will be appreciated, the prosthetic valve 100 can be treated and/or coated with any number of surface or material treatments. Examples of such treatments include, but are not limited to, bioactive agents, including those that modulate thrombosis, those that encourage cellular ingrowth, through growth, and endothelialization, those that resist infection, and those that reduce calcification.

In some embodiments, the prosthetic valve 100 can include one or more structures and/or subcomponents fabricated from a sheet and/or billet, for example, by stamping, drilling, cutting, forging, shearing, machining, etching, and the like.

In some embodiments, the valve frame 102 can include securing members for securing the prosthetic valve 100 to a body lumen, including, but not limited to, hooks, barbs, spikes, protrusions, and the like. The securing members can be disposed on the valve frame 102 and/or stent anchoring frame 104 at or around the exterior or at the proximal end 124 of the stent anchoring frame 104, the distal end 144 of the stent anchoring frame 104, and/or the distal end 134 of the valve frame 102. In some embodiments, the valve frame 102 includes one or more biologically active compounds and/or active chemical entities known in the art, for example, a drug, therapeutic agent, anti-coagulant, anti-proliferant, anti-inflammatory agent, and/or tissue growth modulating agent.

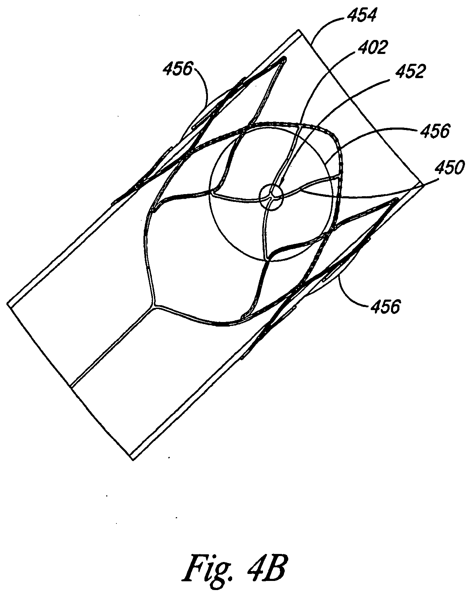

Figures 4A and 4B illustrate an embodiment of a valve frame 402 according to the present disclosure. Figure 4A illustrates a rolled out view of the valve frame 402. As illustrated, the valve frame 402 includes the valve frame members 405 and also additional valve frame members 450. The additional valve frame members 450 can form a valve frame 402 having a bulbous portion 452.

As shown in Figure 4B, the valve frame 402 including the bulbous portion 452 can be formed by heat setting the valve frame 402 over a mandrel 454 that includes a protrusion 456 where the bulbous portion 452 is to be formed. The bulbous portion 452 of the valve frame 402 can provide an increased area, or sinus, between the valve leaflet and the body lumen wall. As one skilled in the art will appreciate, the increased sinus can create increased blood flow behind the valve leaflets when in the closed position, helping to seal the leaflets together when in use.

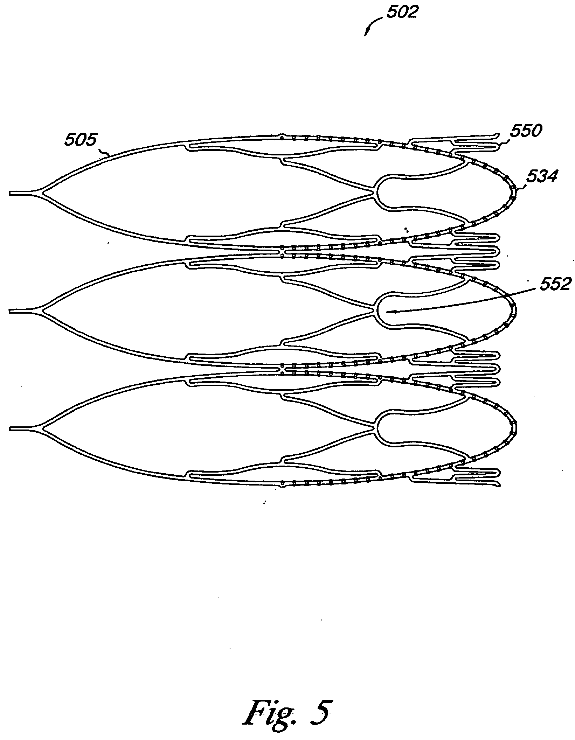

Figure 5 illustrates a rolled out view of an embodiment of a valve frame 502 according to the present disclosure. The valve frame 502 also shows a valve frame 502 that can include a bulbous portion 552. In addition, the valve frame 502 can include the valve frame members 505 and also additional valve frame members 550 near the distal end 534 of the valve frame 502 to provide additional support for the valve frame 502 and to provide additional radial stiffness to secure the valve frame 502 in position when in use.

In other aspects of the present disclosure, delivery devices for delivering a prosthetic valve to a treatment location in a body lumen are provided, as are methods for their use. The delivery devices, or systems, are particularly adapted for use in minimally invasive, interventional procedures, such as percutaneous aortic valve replacements.

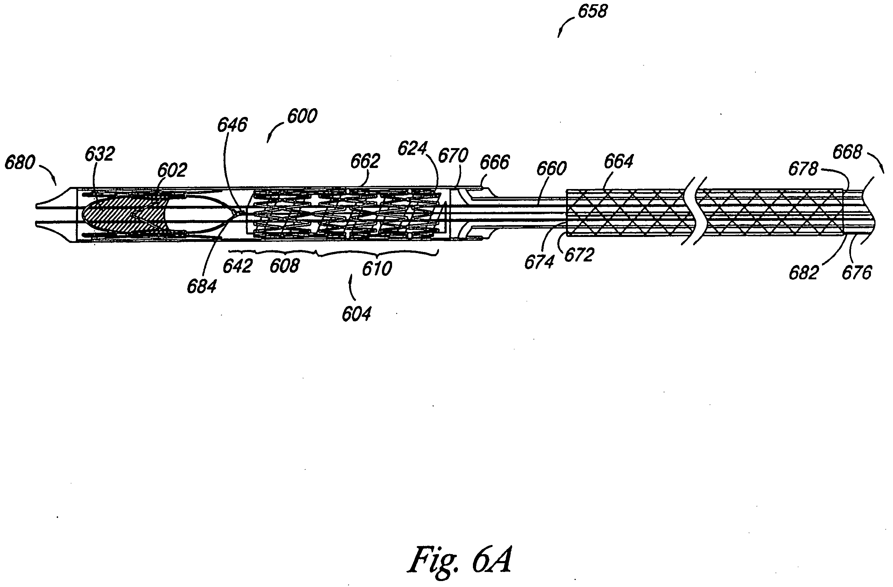

Figures 6A-6E illustrate an example of a system 658 , which does not form part of the present invention. The system 658 includes a prosthetic valve 600, as described herein, releasably joined to an elongate delivery catheter 660, a first retractable sheath 662 positioned around at least a portion of the elongate delivery catheter 660, and a second retractable sheath 664 positioned, for example, proximal to the first retractable sheath 662. The prosthetic valve 600 can be positioned between the elongate delivery catheter 660 and the first retractable sheath 662.

In the examples illustrated in Figures 6A-6E, the elongate delivery catheter 660 can include a catheter lumen extending through the elongate delivery catheter 660. In some embodiments, the catheter lumen can receive a guidewire for guiding the placement of the prosthetic valve 600.

As shown in Figure 6A, the first retractable sheath 662 can be positioned to releasably hold the prosthetic valve 600 in a delivery, or undeployed, state. In some embodiments, the first retractable sheath 662 can have a diameter of about five (5) millimeters (mm). Other dimensions are also possible.

In addition, the first retractable sheath 662 can move longitudinally (e.g., slide) relative the elongate delivery catheter 660 to allow the prosthetic valve 600 to radially expand from its delivery state to its deployed state. In some embodiments, moving the first retractable sheath 662 relative the elongate delivery catheter 660 can be accomplished by pulling a proximal end 666 of the first retractable sheath 662 toward a proximal end 668 of the elongate delivery catheter 660.

In some embodiments, the first retractable sheath 662 can include an inner lining on the inside surface of the first retractable sheath 662. An inner lining can decrease the friction between the prosthetic valve 600 and the first retractable sheath 662 while also sealing the first retractable sheath 662. The inner lining can be formed of, for example, Nylon, Dacron, expanded polytetrafluoroethylene (ePTFE), and/or other materials.

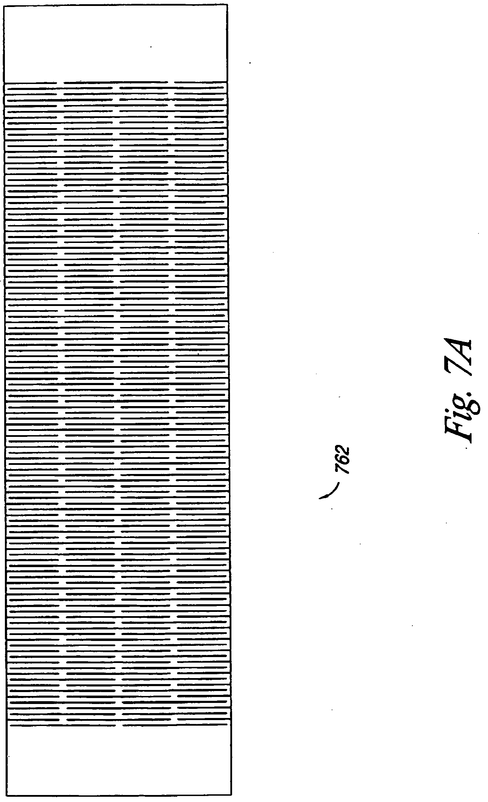

The first retractable sheath 662 can have many possible configurations. For example, in some embodiments, the first retractable sheath 662 can be a flexible tube formed of a metal, metal-alloy, and/or polymers, such as polyvinyl chloride, polyethylene, polyethylene terephalate, polyamide, mixtures, and block-copolymers thereof. Figures 7A-7C illustrate embodiments of the first retractable sheath 762 according to embodiments of the present disclosure. The first retractable sheath 762, as shown, can have a slotted tube configuration.

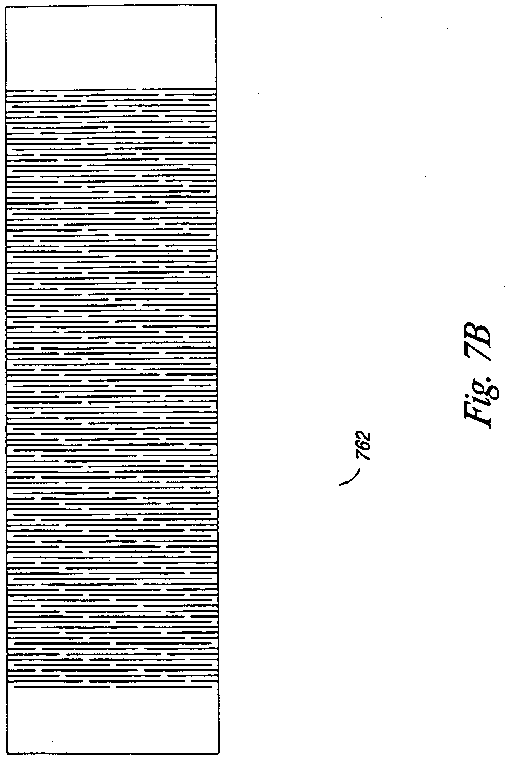

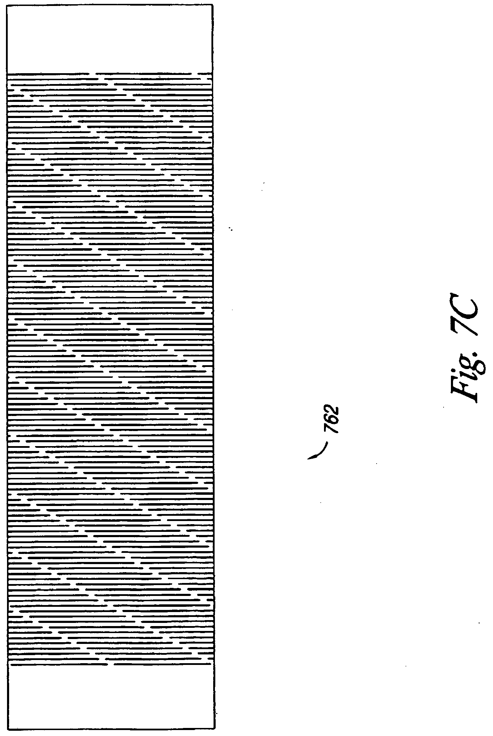

Figure 7A illustrates a first retractable sheath 762 where the slots are offset by a ninety (90) degree angle. Figure 7B illustrates a first retractable sheath 762 where the slots are offset by a forty-five (45) degree angle. Also, Figure 7C illustrates a first retractable sheath 762 where the slots are offset by approximately twelve (12) to thirteen (13) degrees. By having a slotted tube configuration, the flexibility of the first retractable sheath 762 can be modified to the flexibility desired. For example, the first retractable sheath 762 illustrated in Figure 7C can be more flexible than the first retractable sheath 762 illustrated in Figure 7A when formed of the same material with equal dimensions.

Returning to Figures 6A-6E, figure 6B illustrates the system 658 when the first retractable sheath 662 has been partially retracted to allow the valve frame 602 to be radially expanded while holding the first portion 608 and second portion 610 of the stent anchoring frame 604 in the delivery state. As illustrated, the valve frame 602 expands to a deployed state. In such embodiments, the valve frame 602 can expand from, for example, about five (5) mm in diameter to about twenty-five (25) mm in diameter.

In some embodiments, the diameter of the valve frame 602 and/or the stent anchoring frame 604, when deployed, can be provided with a dimension that is from zero (0) to twenty-five (25) percent larger than the dimension of the body lumen. Once deployed, the valve frame 602 and/or the stent anchoring frame 604 can expand the dimension of the body lumen at the treatment location. In this way, the prosthetic valve 600 can reduce the possibility of fluid leakage around the periphery of the prosthetic valve 600. In addition, due to the strength and rigidity of the prosthetic valve 600, the prosthetic valve 600 can have proper apposition to the body lumen to reduce the likelihood of migration of the prosthetic valve 600 once employed.

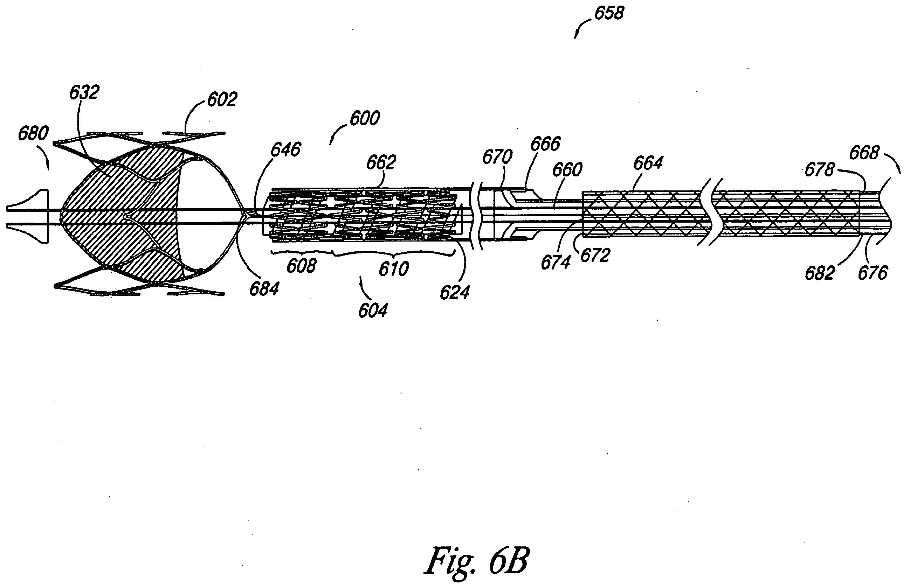

Figure 6B also illustrates that the elongate delivery catheter 660 can include a stopper 670 to hold the prosthetic valve 600 in place when the first retractable sheath 662 is retracted. Also, during this period of partial deployment, the stent anchoring frame 604 can remain constrained by the first retractable sheath 662. Fluid is also able to flow freely through the valve frame 602 and around the delivery system 658.

Figure 6B also illustrates transition zone members 646, as discussed herein, coupling the valve frame 602 and the stent anchoring frame 604. The transition zone members 646 can radially expand to enable the valve frame 602 to fully expand while the first portion 608 of the stent anchoring frame 604 remains constrained. In some embodiments, the transition zone members 646 are formed such that the valve frame 602 can completely deploy.

Once the valve frame 602 is expanded, the position and alignment of the valve frame 602 can be monitored to determine whether it is in a satisfactory position. If the valve frame 602 is not positioned correctly, examples of the present disclosure provide for the repositioning of the prosthetic valve 600.

In someexamples, the second retractable sheath 664 can be positioned proximal to the first retractable sheath 662. The second retractable sheath 664 can be delivered to the treatment site in a delivery state, and can be expanded when the valve frame 602 has been deployed to reposition the valve frame 602. In some embodiments, the second retractable sheath 664 can be a wallstent, where the distal and proximal ends of the wallstent are pushed towards each other to expand the diameter of the wallstent.

In various examples, the second retractable sheath 664 can be heat set in the delivery state, as shown in Figure 6B, where the second retractable sheath 664 can return to the delivery state when the second retractable sheath 664 is not under a strain and/or stress.

For example, the second retractable sheath 664 can include cables 672 attached to the distal end 674 of the second retractable sheath 664 and an expandable member 676 coupled to the proximal end 678 of the second retractable sheath 664. The cables 672 can be pulled toward the proximal end 668 of the elongate delivery catheter 660 while the expandable member 676 can be pushed toward the distal end 680 of the elongate delivery catheter 660. In some examples, the expandable member 676 can include fingers 682 coupled to the proximal end 678 of the second retractable sheath 664 that can separate as the second retractable sheath 664 expands.

In some examples, the second retractable sheath 664 can be heat set in an expanded state, as discussed herein, where the second retractable sheath 664 can be delivered under strain using an outer sheath, and can be expanded by retracting the outer sheath.

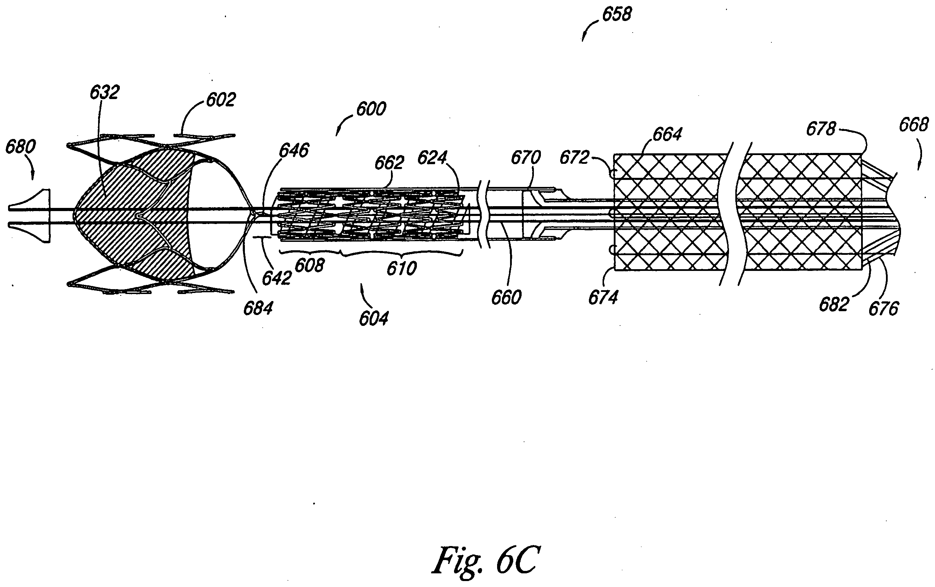

Figure 6C illustrates the system 658 when the second retractable sheath 664 has been expanded. As shown, the second retractable sheath 664 can expand to a diameter that allows the second retractable sheath 664 to be advanced over the first retractable sheath 662 and the stopper 670 associated with the first retractable sheath
662. In some examples, the second retractable sheath 664 can expand from about one (1) to about five (5) mm to a diameter in a range of about ten (10) to about twenty (20) mm.

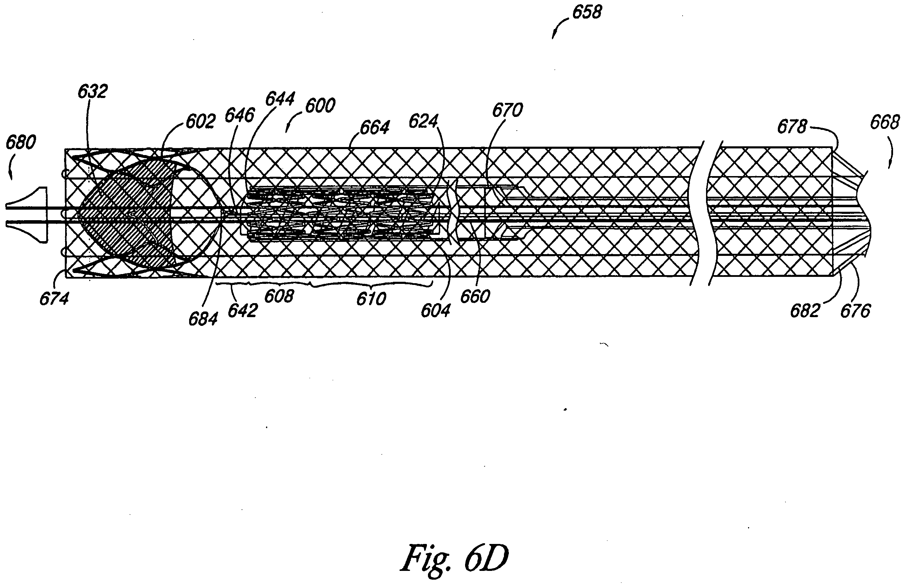

As shown in Figure 6C, the first retractable sheath 662 can be positioned over the first portion 608 and second portion 610 of the stent anchoring frame 604. In some examples, the first retractable sheath 662 can be retracted further to allow the first portion 608 of the stent anchoring frame to partially expand. The second retractable sheath 664 can then be advanced over the contiguous surface of the first portion 608 of the stent anchoring frame 604 and/or the valve frame 602 to compress the prosthetic valve 600, as shown in Figure 6D. In this way, the valve frame 602 diameter is decreased to enable the repositioning of the valve frame 602.

Figure 6D illustrates the system 658 when the second retractable sheath 664 has been advanced over the valve frame 602 and/or the first portion 608 of the stent anchoring frame 604 to contract the valve frame 602 and the first portion 608 of the stent anchoring frame 604.

In some embodiments, the second retractable sheath 664 can be formed of ring structure, where a number of expandable rings are connected by a flexible tube. The expandable rings can be delivered with a diameter of about five (5) mm and then expand to a diameter of about ten (10) mm to enable the second sheath to function as a repositioning device. In some embodiments, the second retractable sheath 664 can be lined with a low friction surface that can easily slide over the first portion 608 of the stent anchoring frame 604 and/or the valve frame 602, for example, ePTFE.

As illustrated in Figure 6D, in some examples, to enable the second sheath 664 to re-contract the valve frame 602 and/or the first portion 608 of the stent anchoring frame 604 without pushing the valve frame 602 toward the distal end 680 of the elongate delivery catheter 660, the elongate delivery catheter 660 can include a cable member 684, or cable members 684, to hold the prosthetic valve 600 in place when the second retractable sheath 664 is moved toward the distal end 680 of the elongate delivery catheter 660. The cable members 684 can be positioned at a number of different places, for example, at the transition zone 642 of the valve frame 602 as shown, the first portion 608 of the stent anchoring frame 604, the distal end 644 of the stent anchoring frame 604, and/or the proximal end 624 of the stent anchoring frame 604, among other locations.

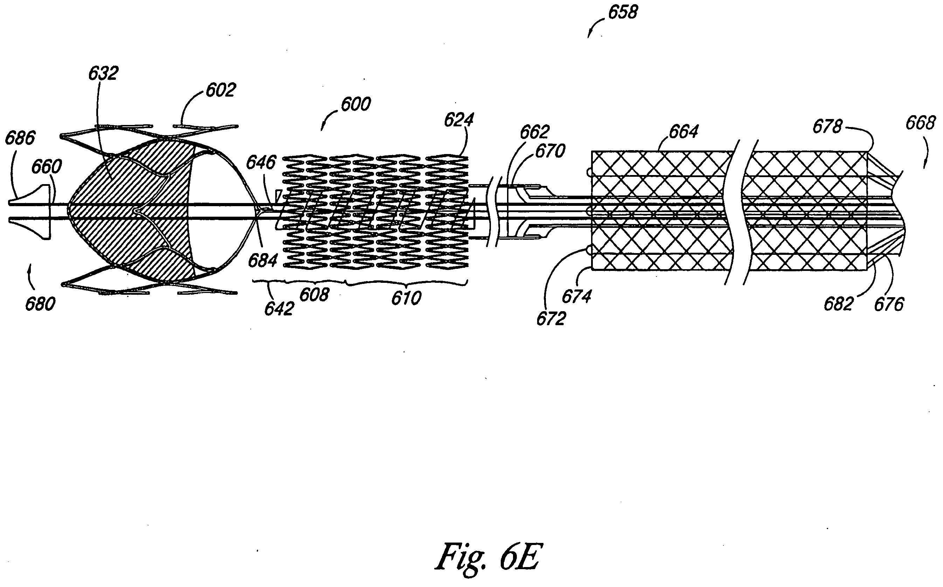

Once the prosthetic valve 600 is re-constrained in the second sheath 664, the prosthetic valve 600 can be repositioned and the second sheath 664 can be retracted to re-deploy the valve frame 602. This process can be repeated until the position, stability, and functioning of the valve frame 602 is satisfactory. Once the valve frame 602 is in a satisfactory position, the second retractable sheath 664 and the first retractable sheath 662 can be retracted to allow the prosthetic valve 600 to fully expand, as illustrated in Figure 6E.

To remove the system 658, the second retractable sheath 664 can be contracted by releasing the expandable member 676 coupled to the proximal end 678 of the second retractable sheath and the cables 672 holding the second retractable sheath 664 in place. Once the second retractable sheath 664 returns to the delivery state, the system 658 can be removed.

In examples where the second retractable sheath 664 is delivered with an outer sheath to constrain the second retractable sheath, the system 658 can be removed by contracting the second retractable sheath 664 by pulling the expandable member 676 coupled to the proximal end 678 of the second retractable sheath toward the proximal end 668 of the elongate delivery catheter 660 while the cables 672 hold the second retractable sheath 664 in place. Once the second retractable sheath 664 is contracted, the system 658 can be removed.

In some examples, the system 658 accomplishes the repositioning of the prosthetic valve 600 by sliding the first retractable sheath 664 over the deployed valve frame 602 rather than including a second retractable sheath 664. In such examples, the transition zone members 646 can be formed with an increased strength to hold the valve frame 602 in place while the valve frame 602 is contracted from a diameter of about twenty-five (25) mm to about five (5) mm.

In some examples, the elongate delivery catheter 660 can include a distal tip 686. The distal tip 686 can have a conical configuration, where the tip diameter decreases in size to a point at the distal end 680 of the elongate delivery catheter 660. For example, the system 658 can have a five (5) mm diameter at the prosthetic valve 600, and decrease to a two (2) mm diameter at the distal end 680 of the elongate delivery catheter 660.

As discussed herein, the prosthetic valve 600 can be formed of a self-expandable material or a material with a spring bias, where the prosthetic valve 600 can expand when the first retractable sheath 662 and/or second retractable sheath 664 has been removed. In some examples, an expandable balloon can be included to secure the prosthetic valve 600 inside a body lumen.

In some examples, the expandable balloon can be a perfusion balloon. A perfusion balloon can be used to radially expand the valve frame 602 while allowing fluid, for example, blood, to pass through the delivery catheter 660 and prosthetic valve 600 while the prosthetic valve 600 is being positioned in the vasculature.

The delivery catheter 660 can be formed of a number of materials. Materials include polymers, such as PVC, PE, PET, polyamide, mixtures, and block copolymers thereof, as discussed herein with respect to the first retractable sheath 662. In addition, each of the delivery catheter 660, the first retractable sheath 662, and/or the second retractable sheath 664 can have a wall thickness and an inner diameter sufficient to allow the structures to slide longitudinally relative each other, as described herein, and to maintain the prosthetic valve 600 in a delivery state, as discussed herein.

In an additional embodiment, the prosthetic valve 600 can further include a sealing material positioned on the periphery of the valve frame 602. In one embodiment, once implanted, the sealing material can swell due the presence of liquid to occupy volume between the valve frame 602 and the tissue on which the prosthetic valve 600 has been implanted so as to prevent leakage of the liquid around the outside of the prosthetic valve 600.

A variety of suitable materials for the sealing material are possible. For example, the sealing material can be selected from the general class of materials that include polysaccharides, proteins, and biocompatible gels. Specific examples of these polymeric materials can include, but are not limited to, those derived from poly(ethylene oxide) (PEO), poly(ethylene glycol) (PEG), poly(vinyl alcohol) (PVA), poly(vinylpyrrolidone) (PVP), poly(ethyloxazoline) (PEOX) polyaminoacids, pseudopolyamino acids, and polyethyloxazoline, as well as copolymers of these with each other or other water soluble polymers or water insoluble polymers. Examples of the polysaccharide include those derived from alginate, hyaluronic acid, chondroitin sulfate, dextran, dextran sulfate, heparin, heparin sulfate, heparan sulfate, chitosan, gellan gum, xanthan gum, guar gum, water soluble cellulose derivatives, and carrageenan. Examples of proteins include those derived from gelatin, collagen, elastin, zein, and albumin, whether produced from natural or recombinant sources.

In some embodiments, the stent anchoring frame 604 can include a sealing band surrounding the stent anchoring frame 604 to prevent leakage of liquid around the outside of the prosthetic valve 600. The sealing band may comprise a relatively soft biocompatible material, such as polyurethane or other polymer. In some embodiments, the sealing band can be porous or is otherwise capable of expanding or swelling when exposed to fluids, thereby enhancing the ability of the sealing band.

The sealing band may include a functional composition such as an adhesive, a fixative, or therapeutic agent such as a drug or other materials.

The embodiments of the prosthetic valve 600 described herein may be used to replace, supplement, or augment valve structures within one or more lumens of the body. For example, embodiments of the present invention may be used to replace an incompetent cardiac valve of the heart, such as the aortic and/or pulmonary valves of the heart. In one embodiment, the native cardiac valve can either remain in place or be removed (e.g., via a valvoplasty procedure) prior to implanting the cardiac valve of the present disclosure.

In addition, positioning the system 658 having the prosthetic valve 600 as discussed herein includes introducing the system into the cardiovascular system of the patient using minimally invasive percutaneous, transluminal techniques. For example, a guidewire can be positioned within the cardiovascular system of a patient that includes the predetermined location. The system 658 of the present disclosure, including the prosthetic valve 600 as described herein, can be positioned over the guidewire and the system 658 advanced so as to position the prosthetic valve 600 at or adjacent the predetermined location. In one example, radio opaque markers on the catheter 660 and/or the prosthetic valve 600, as described herein, can be used to help locate and position the prosthetic valve 600.

The prosthetic valve 600 can be deployed from the system at the predetermined location in any number of ways, as described herein. In one embodiment, the prosthetic valve 600 of the present disclosure can be deployed and placed in any number of cardiovascular locations. For example, the prosthetic valve 600 can be deployed and placed within a major artery of a patient. In one embodiment, major arteries include, but are not limited to, the aorta. In addition, valves of the present invention can be deployed and placed within veins. Other locations are also possible.

Once implanted, the prosthetic valve 600 can provide sufficient contact with the body lumen wall to allow the leaflets 632 to prevent retrograde flow between the prosthetic valve 600 and the body lumen wall. In addition, the stent anchoring frame 604 can securely locate the prosthetic valve 600 and prevent migration of the prosthetic valve 600. The prosthetic valve 600 described herein also displays sufficient flexibility and resilience so as to accommodate changes in the body lumen diameter, while maintaining the proper placement of prosthetic valve 600. As described herein, the prosthetic valve 600 can engage the lumen so as to reduce the volume of retrograde flow through and around prosthetic valve 600. It is, however, understood that some leaking or fluid flow may occur between the valve frame 602 and the body lumen and/or through valve leaflets 632.

While the present invention has been shown and described in detail above, it will be clear to the person skilled in the art that changes and modifications may be made without departing from the spirit and scope of the invention. As such, that which is set forth in the foregoing description and accompanying drawings is offered by way of illustration only and not as a limitation. The actual scope of the invention is intended to be defined by the following claims, along with the full range of equivalents to which such claims are entitled. In addition, one of ordinary skill in the art will appreciate upon reading and understanding this disclosure that other variations for the invention described herein can be included within the scope of the present invention.

In the foregoing Detailed Description, various features are grouped together in several embodiments for the purpose of streamlining the disclosure. This method of disclosure is not to be interpreted as reflecting an intention that the embodiments of the invention require more features than are expressly recited in each claim. Rather, as the following claims reflect, inventive subject matter lies in less than all features of a single disclosed embodiment. Thus, the following claims are hereby incorporated into the Detailed Description, with each claim standing on its own as a separate embodiment.

## Claims

1. A percutaneous prosthetic heart valve (100, 600), comprising:
an expandable valve frame (102, 402, 502, 602) including valve frame members (105, 405, 450, 505, 550);
a valve leaflet (132, 632) coupled to the valve frame (102, 402, 502, 602); and
an expandable stent anchoring frame (104, 204, 304, 604) coupled to the valve frame (102, 402, 502, 602) including stent frame members (106, 206, 306) defining a first portion (108, 308, 608) and a second portion (110, 210, 310, 610) having greater flexibility than the first portion (108, 308, 608), where the first portion (108, 308, 608) and the valve frame (102, 402, 502, 602) define a length (140), and where the stent frame members (106, 206, 306) and the valve frame members (105, 405, 450, 505, 550) along the length (140) provide a contiguous surface over which a delivery device (662, 762) can repeatedly slide over the length (140) in its entirety in two longitudinal directions when the first portion (108, 308, 608) and the valve frame (102, 402, 502, 602) are in a partially expanded state during delivery from the delivery device (662, 762),
**characterized in that**
the stent frame members (106, 206, 306) are annular stent frame members (106, 206, 306), having apexes (114, 314) and connectors (112, 312) coupling the annular stent frame members (106, 206, 306), where in the first portion (108, 308, 608) each apex (114, 314) formed by the annular frame member is connected by a connector (112, 312) to an apex of an adjacent stent frame member (106, 206, 306) and in the second portion (110, 210, 310, 610) not every apex formed by the annular frame member is connected to an apex of an adjacent stent frame member.

2. The valve (100, 600) of any of the preceding claims, where the valve frame (102, 402, 502, 602) includes a transition zone (142, 642), where the transition zone (142, 642) is positioned at a proximal end of the valve frame (102, 402, 502, 602), and
where the transition zone (142, 642) includes transition zone members (146, 646) coupled to a distal end (144, 644) of the stent anchoring frame (104, 204, 304, 604).

3. The valve (100, 600) of any of the preceding claims, where the transition zone members (146, 646) are coupled to the first portion (108, 308, 608) of the stent anchoring frame (104, 204, 304, 604).

4. The valve (100, 600) of any of the preceding claims, where the valve frame (102, 402, 502, 602) includes a support ring (148) at the distal end (134, 534) of the valve frame (102, 402, 502, 602).

5. The valve (100, 600) of any of the preceding claims, where the second portion (110, 210, 310, 610) of the stent anchoring frame (104, 204, 304, 604) is positioned proximal to the first portion (108, 308, 608) of the stent anchoring frame (104, 204, 304, 604) in a longitudinal direction.

6. A method, comprising:
forming an expandable valve frame (102, 402, 502, 602) including valve frame members (105, 405, 450, 505, 550); coupling a valve leaflet (132, 632) to the valve frame (102, 402, 502, 602); forming a stent anchoring frame (104, 204, 304, 604) including stent frame members (106, 206, 306) defining a first portion (108, 308, 608) and a second portion (110, 210, 310, 610) having greater flexibility than the first portion (108, 308, 608); and
coupling the stent anchoring frame (104, 204, 304, 604) to the valve frame (102, 402, 502, 602), where the first portion (108, 308, 608) and the valve frame (102, 402, 502, 602) define a length (140), and where the stent frame members (106, 206, 306) and the valve frame members (105, 405, 450, 505, 550) along the length (140) provide a contiguous surface over which a retractable sheath (662, 762) can repeatedly slide longitudinally over the length (140) in its entirety in two directions when the first portion (108, 308, 608) and the valve frame (102, 402, 502, 602) are in a partially expanded state during delivery from a delivery device (662, 762),
**characterized in that**
the stent frame members (106, 206, 306) are annular stent frame members (106, 206, 306), having apexes (114, 314) and connectors (112, 312) coupling the annular stent frame members (106, 206, 306), where in the first portion (108, 308, 608) each apex (114, 314) formed by the annular frame member is connected by a connector to an apex of an adjacent stent frame member (106, 206, 306) and in the second portion (110, 210, 310, 610) not every apex formed by the annular frame member is connected to an apex of an adjacent stent frame member.

7. The method of any of the preceding claims, where the method includes positioning the valve frame (102, 402, 502, 602) coupled to the stent anchoring frame (104, 204, 304, 604) on the delivery device (662, 762) between an elongate delivery catheter (660) and the retractable sheath (662, 762), where the retractable sheath (662, 762) moves longitudinally relative the elongate delivery catheter (660).

## Patentansprüche

1. Perkutan zuführbare Herzklappenprothese (100, 600), die umfasst:
einen expandierbaren Klappen-Rahmen (102, 402, 502, 602), der Klappen-Rahmen-Elemente (105, 405, 450, 505, 550) beinhaltet;
ein Klappen-Segel (132, 632), das mit dem Klappen-Rahmen (102, 402, 502, 602) verbunden ist; und
einen expandierbaren Stent-Verankerungs-Rahmen (104, 204, 304, 604), der mit dem Klappen-Rahmen (102, 402, 502, 602) verbunden ist, der Stent-Rahmen-Elemente (106, 206, 306) beinhaltet, die einen ersten Bereich (108, 308, 608) und einen zweiten Bereich (110, 210, 310, 610), der eine größere Flexibilität als der erste Bereich (108, 308, 608) aufweist, definieren, wobei der erste Bereich (108, 308, 608) und der Klappen-Rahmen (102, 402, 502, 602) ein Länge (140) definieren, und wobei die Stent-Rahmen-Elemente (106, 206, 306) und die Klappen-Rahmen-Elemente (105, 405, 450, 505, 550) entlang der Länge eine zusammenhängende Oberfläche bilden, über die eine Zuführvorrichtung (662, 762) wiederholt über die Gesamtheit der Länge (140) in zwei longitudinale Richtungen gleiten kann, wenn der erste Bereich (108, 308, 608) und der Klappen-Rahmen (102, 402, 502, 602) während der Zuführung von der Zuführvorrichtung (662, 762) in einem teilweise expandierten Zustand sind,
**gekennzeichnet dadurch, dass**
die Stent-Rahmen-Elemente (106, 206, 306) ringförmige Stent-Rahmen-Elemente (106, 206, 306) mit Apizes (114, 314) und Konnektoren (112, 312), die die ringförmige Stent-Rahmen-Elemente (106, 206, 306) verbinden, sind, wobei in dem ersten Bereich (108, 308, 608) jeder Apex (114, 314), der durch das ringförmige Rahmen-Element gebildet wird, durch einem Konnektor (112, 312) mit einem Apex eines benachbarten Stent-Rahmen-Elements (106, 206, 306) verbunden ist und wobei in dem zweiten Bereich (110, 210, 310, 610) nicht jeder Apex (114, 314), der durch das ringförmige Rahmen-Element gebildet wird, mit einem Apex eines benachbarten Stent-Rahmen-Elements verbunden ist.

2. Klappe (100, 600) gemäß irgendeinem der vorangehenden Ansprüche, wobei der Klappen-Rahmen (102, 402, 502, 602) eine Übergangszone (142, 642) beinhaltet, wobei die Übergangszone (142, 642) an einem proximalen Ende des Klappen-Rahmens (102, 402, 502, 602) positioniert ist und wobei die Übergangszone (142, 642) Übergangszonen-Elemente (146, 646) umfasst, die mit einem distalen Ende des Stent-Verankerungs-Rahmen (104, 204, 304, 604) verbunden sind.

3. Klappe (100, 600) gemäß irgendeinem der vorangehenden Ansprüche, wobei die Übergangszonen-Elemente (146, 646) mit dem ersten Bereich (108, 308, 608) des Stent-Verankerungs-Rahmens (104, 204, 304, 604) verbunden sind.

4. Klappe (100, 600) gemäß irgendeinem der vorangehenden Ansprüche, wobei der Klappen-Rahmen (102, 402, 502, 602) einen Stützring (148) an dem distalen Ende (134, 534) des Klappen-Rahmens (102, 402, 502, 602) beinhaltet.

5. Klappe (100, 600) gemäß irgendeinem der vorangehenden Ansprüche, wobei der zweite Bereich (110, 210, 310, 610) des Stent-Verankerungs-Rahmens (104, 204, 304, 604) proximal des ersten Bereichs (108, 308, 608) des Stent-Verankerungs-Rahmens (104, 204, 304, 604) in einer longitudinale Richtung positioniert ist.

6. Verfahren, umfassend:
Bilden eines expandierbaren Klappen-Rahmens (102, 402, 502, 602), der Klappen-Rahmen-Elemente (105, 405, 450, 505, 550) beinhaltet;
Koppeln eines Klappen-Segels (132, 632) an den Klappen-Rahmen (102, 402, 502, 602);
Bilden eines Stent-Verankerungs-Rahmens (104, 204, 304, 604), der Stent-Rahmen-Elemente (106, 206, 306) beinhaltet, die einen ersten Bereich (108, 308, 608) und einen zweiten Bereich (110, 210, 310, 610), der eine größere Flexibilität als der erste Bereich (108, 308, 608) aufweist, definieren; und
Koppeln des Stent-Verankerungs-Rahmens (104, 204, 304, 604) an den Klappen-Rahmen (102, 402, 502, 602), wobei der erste Bereich (108, 308, 608) und der Klappen-Rahmen (102, 402, 502, 602) eine Länge (140) definieren, und wobei die Stent-Rahmen-Elemente (106, 206, 306) und die Klappen-Rahmen-Elemente (105, 405, 450, 505, 550) entlang der Länge eine zusammenhängende Oberfläche bilden, über die eine rückziehbare Hülle (662, 762) über die Gesamtheit der Länge (140) in zwei longitudinale Richtungen wiederholt gleiten kann, wenn der erste Bereich (108, 308, 608) und der Klappen-Rahmen (102, 402, 502, 602) während der Zuführung von einer Zuführvorrichtung (662, 762) in einem teilweise expandierten Zustand sind,
**gekennzeichnet dadurch, dass**
die Stent-Rahmen-Elemente (106, 206, 306) ringförmige Stent-Rahmen-Elemente (106, 206, 306) mit Apizes (114, 314) und Konnektoren (112, 312), die die ringförmige Stent-Rahmen-Elemente (106, 206, 306) verbinden, sind, wobei in dem ersten Bereich (108, 308, 608) jeder Apex (114, 314), der durch das ringförmige Rahmen-Element gebildet wird, mit einem Konnektor (112, 312) mit einem Apex eines benachbarten Stent-Rahmen-Elements (106, 206, 306) verbunden ist und wobei in dem zweiten Bereich (110, 210, 310, 610) nicht jeder Apex (114, 314), der durch das ringförmige Rahmen-Element gebildet wird, mit einem Apex eines benachbarten Stent-Rahmen-Elements verbunden ist.

7. Verfahren gemäß irgendeinem der vorangehenden Ansprüche, wobei das Verfahren Positionieren des Klappen-Rahmens (102, 402, 502, 602), der mit dem Stent-Verankerungs-Rahmens (104, 204, 304, 604) auf dem Zuführgerät (662, 762) verbunden ist, zwischen einem länglichen Zuführkatheter (660) und der rückziehbaren Hülle (662, 762) beinhaltet, wobei die rückziehbare Hülle (662, 762) sich longitudinal relativ zu dem länglichen Zuführkatheter (660) bewegt.

## Revendications

1. Valve cardiaque prothétique percutanée (100, 600), comprenant :
une armature de valve dilatable (102, 402, 502, 602) comprenant des éléments d'armature de valve (105, 405, 450, 505, 550) ;
un feuillet de valve (132, 632) accouplé à l'armature de valve (102, 402, 502, 602) ; et
une structure de fixation formant stent dilatable (104, 204, 304, 604) accouplée à l'armature de valve (102, 402, 502, 602) comprenant des éléments de structure formant stent (106, 206, 306) définissant une première partie (108, 308, 608) et une seconde partie (110, 210, 310, 610) présentant une souplesse supérieure à celle de la première partie (108, 308, 608), la première partie (108, 308, 608) et l'armature de valve (102, 402, 502, 602) définissant une longueur (140), et les éléments de structure formant stent (106, 206, 306) et les éléments d'armature de valve (105, 405, 450, 505, 550) le long de la longueur (140) formant une surface contiguë sur laquelle peut glisser, de manière répétée, un dispositif d'implantation (662, 762) sur l'intégralité de la longueur (140) dans deux directions longitudinales lorsque la première partie (108, 308, 608) et l'armature de valve (102, 402, 502, 602) se trouvent dans un état partiellement dilaté lors de l'implantation depuis le dispositif d'implantation (662, 762),
**caractérisée en ce que**
les éléments de structure formant stent (106, 206, 306) sont des éléments de structure formant stent (106, 206, 306) annulaires, comportant des pointes (114, 314) et des raccords (112, 312) accouplant les éléments de structure formant stent (106, 206, 306) annulaires, chaque pointe (114, 314) formée par l'élément de structure annulaire, dans la première partie (108, 308, 608), étant raccordée par un raccord (112, 312) à une pointe d'un élément de structure formant stent (106, 206, 306) adjacent et les pointes formées par l'élément de structure annulaire, dans la seconde partie (110, 210, 310, 610), n'étant pas toutes raccordées à une pointe d'un élément de structure formant stent adjacent.

2. Valve (100, 600) selon l'une quelconque des revendications précédentes, dans laquelle l'armature de valve (102, 402, 502, 602) comprend une zone de transition (142, 642), dans laquelle la zone de transition (142, 642) est positionnée à une extrémité proximale de l'armature de valve (102, 402, 502, 602), et dans laquelle la zone de transition (142, 642) comprend des éléments de zone de transition (146, 646) accouplés à une extrémité distale (144, 644) de la structure de fixation formant stent (104, 204, 304, 604).

3. Valve (100, 600) selon l'une quelconque des revendications précédentes, dans laquelle les éléments de zone de transition (146, 646) sont accouplés à la première partie (108, 308, 608) de la structure de fixation formant stent (104, 204, 304, 604).

4. Valve (100, 600) selon l'une quelconque des revendications précédentes, dans laquelle l'armature de valve (102, 402, 502, 602) comprend un anneau de support (148) à l'extrémité distale (134, 534) de l'armature de valve (102, 402, 502, 602).

5. Valve (100, 600) selon l'une quelconque des revendications précédentes, dans laquelle la seconde partie (110, 210, 310, 610) de la structure de fixation formant stent (104, 204, 304, 604) est en position proximale vis-à-vis de la première partie (108, 308, 608) de la structure de fixation formant stent (104, 204, 304, 604) dans une direction longitudinale.

6. Procédé, comprenant :
former une armature de valve dilatable (102, 402, 502, 602) comprenant des éléments d'armature de valve (105, 405, 450, 505, 550) ; accoupler un feuillet de valve (132, 632) à l'armature de valve (102, 402, 502, 602) ; former une structure de fixation formant stent (104, 204, 304, 604) comprenant des éléments de structure formant stent (106, 206, 306) définissant une première partie (108, 308, 608) et une seconde partie (110, 210, 310, 610) présentant une souplesse supérieure à celle de la première partie (108, 308, 608) ; et
accoupler la structure de fixation formant stent (104, 204, 304, 604) à l'armature de valve (102, 402, 502, 602), la première partie (108, 308, 608) et l'armature de valve (102, 402, 502, 602) définissant une longueur (140), et les éléments de structure formant stent (106, 206, 306) et les éléments d'armature de valve (105, 405, 450, 505, 550) le long de la longueur (140) formant une surface contiguë sur laquelle peut glisser longitudinalement, de manière répétée, une gaine rétractable (662, 762) sur l'intégralité de la longueur (140) dans deux directions lorsque la première partie (108, 308, 608) et l'armature de valve (102, 402, 502, 602) se trouvent dans un état partiellement dilaté lors de l'implantation depuis un dispositif d'implantation (662, 762), **caractérisé en ce que**
les éléments de structure formant stent (106, 206, 306) sont des éléments de structure formant stent (106, 206, 306) annulaires, comportant des pointes (114, 314) et des raccords (112, 312) accouplant les éléments de structure formant stent (106, 206, 306) annulaires, chaque pointe (114, 314) formée par l'élément de structure annulaire, dans la première partie (108, 308, 608), étant raccordée par un raccord à une pointe d'un élément de structure formant stent (106, 206, 306) adjacent et les pointes formées par l'élément de structure annulaire, dans la seconde partie (110, 210, 310, 610), n'étant pas toutes raccordées à une pointe d'un élément de structure formant stent adjacent.

7. Procédé selon l'une quelconque des revendications précédentes, le procédé comprenant l'étape consistant à positionner l'armature de valve (102, 402, 502, 602) accouplée à la structure de fixation formant stent (104, 204, 304, 604) sur le dispositif d'implantation (662, 762) entre un cathéter d'implantation allongé (660) et la gaine rétractable (662, 762), la gaine rétractable (662, 762) se déplaçant longitudinalement par rapport au cathéter d'implantation allongé (660).
